# EUROPEAN PATENT APPLICATION

(11) **EP 2 601 936 A1**
(43) Date of publication of application: **12.06.2013**
(21) Application number: 11814681.0
(22) Date of filing: 03.08.2011
(51) Int. Cl.: A61K 9/20, A61K 31/4439, A61K 47/26, A61K 47/38, A61P 1/04

(54) **COMPRESSED COMPOSITION**

(30) Priority: 03.08.2010 JP 2010174782
(71) Applicant: Eisai R&D Management Co., Ltd., Tokyo 112-8088 (JP)
(72) Inventor: SAKAMOTO, Hirokazu, Kakamigahara-shi Gifu 501-6195 (JP); NAGANE, Kentaro, Kakamigahara-shi Gifu 501-6195 (JP); NOHARA, Masami, Kakamigahara-shi Gifu 501-6195 (JP)
(74) Representative: HOFFMANN EITLE
(86) International application number: PCT/JP2011/067806
(87) International publication number: WO 2012/018056

(57) **Abstract**

An object of the present invention is to provide a compressed composition that is superior in terms of maintaining the function of a film or in terms of content uniformity even during tableting process.

The present invention provides a compressed composition, comprising: coated granules containing a physiologically active compound or pharmacologically acceptable salt thereof, and an excipient, wherein the excipient contains at least one selected from the group consisting of low-substituted hydroxypropylcellulose, lactose and crystalline cellulose, and the total amount thereof is 0.5 parts by weight or more in 1 part by weight of the excipient, and the excipient is physically mixed with or coated onto the coated granules followed by compressing at low pressure.

## Description

### Technical Field

The present invention relates to a compressed composition. More particularly, the present invention relates to a compressed composition comprising coated granules and an excipient and obtained by compressing at low pressure.

### Cross-Reference to Related Application(s)

The present application is based on a Japanese patent application (Japanese Patent Application No. 2010-174782) filed on August 3, 2010, the contents of which are incorporated herein by reference.

### Background Art

A matrix tablet is known as a pharmaceutical preparation obtained by tableting.
Matrix tablets are tablets obtained by mixing a principal agent or granules containing principal-agent with an excipient and then tableting after granulation as necessary.
In order to prevent destruction of the coating film of granules during tableting of the matrix tablets, normally the tablets are produced by adding excipients, the amount of which is equal to or greater than the amount of a principal agent or granules containing principal-agent. Thus, there is susceptibility to the occurrence of the problem that tablets size become excessively large (see Non-Patent Documents 1 and 2). In addition, it is necessary to compress by lower pressure in order to prevent destruction of the coating film of granules, thereby requiring excipients that have superior compression ability.
In addition, in the case the particle diameter differs between the principal agent or granules containing principal-agent and the excipients, there is also the problem of segregation (content uniformity) between the principal agent or granules containing principal-agent and excipients (see Non-Patent Document 3).

### Prior Art Document

### Non-Patent Document

Non-Patent Document 1: Chem. Pharm. Bull. 51 (8), p. 942-947 (2003)
Non-Patent Document 2: Chem. Pharm. Bull. 46(5), p. 826-830 (1998)
Non-Patent Document 3: Powder Technology, Asakura Publishing Co., Ltd., p. 221

### Summary of the Invention

### Problem to be solved by the Invention

An object of the present invention is to provide a compressed composition that is superior in terms of maintaining the function of a film or in terms of content uniformity even during tableting process.

### Means for Solving the Problem

As a result of extensive studies in consideration of the above-mentioned circumstances, the inventors of the present invention found that a compressed composition can be produced that is superior in terms of maintaining the function of a film or in terms of content uniformity by physically mixing or coating an excipient containing at least one selected from the group of low-substituted hydroxypropylcellulose, lactose and crystalline cellulose with or onto coated granules followed by compressing at low pressure, thereby leading to completion of the present invention.

Namely, the present invention is as described below.
[1] A compressed composition, comprising:
   coated granules containing a physiologically active compound or pharmacologically acceptable salt thereof, and an excipient, wherein
   the excipient contains at least one selected from the group consisting of low-substituted hydroxypropylcellulose, lactose and crystalline cellulose, and the total amount thereof is 0.5 parts by weight or more in 1 part by weight of the excipient, and
   the excipient is physically mixed with or coated onto the coated granules followed by compressing at low pressure.
[2] The compressed composition described in [1], wherein the excipient is physically mixed with the coated granules followed by compressing at a low pressure from 0.5 kN to 6 kN.
[3] The compressed composition described in [1], wherein the excipient is coated onto the coated granules followed by compressing at a low pressure from 0.5 kN to 6 kN.
[4] The compressed composition described in any of [1] to [3], wherein the coated granules have:
   a core containing a physiologically active compound or pharmacologically acceptable salt thereof, and
   an enteric film, a sustained-release film, a pulsatile-release film, a gastric film, a water-insoluble film for bitter taste masking, smell masking or light masking, or a water-soluble film coated onto the core.
[5] The compressed composition described in any of [1] to [4], wherein the core has:
   a core substance, and
   a principal agent layer coated onto the core substance and containing a physiologically active compound or pharmacologically acceptable salt thereof.
[6] The compressed composition described in any of [1] to [5], wherein the physiologically active compound is a benzimidazole compound.
[7] The compressed composition described in any of [1] to [6], wherein the excipient further contains a sugar or a sugar-alcohol.
[8] The compressed composition described in any of [1] to [7], wherein the excipient further contains a glidant.
[9] The compressed composition described in any of [1] to [8], wherein the excipient further contains a lubricant.
[10] The compressed composition described in [2], containing 1 part by weight of the coated granules and from 0.1 parts by weight to 10 parts by weight of the excipient.
[11] The compressed composition described in [3], containing 1 part by weight of the coated granules and from 0.1 parts by weight to 1 part by weight of the excipient.
[12] The compressed composition described in any of [1] to [11], which is a matrix tablet.
[13] A dry coated tablet containing the matrix tablet described in [12] as the inner core thereof.
[14] A method for producing a compressed composition, comprising the steps of:
   physically mixing an excipient and coated granules containing a physiologically active compound or pharmacologically acceptable salt thereof, or coating an excipient onto coated granules, and
   compressing the resulting product at low pressure, wherein
   the excipient contains at least one selected from the group consisting of low-substituted hydroxypropylcellulose, lactose and crystalline cellulose, and the total amount thereof is 0.5 parts by weight or more in 1 part by weight of the excipient.

### Effect of the Invention

According to the present invention, a compressed composition can be provided that is superior in terms of maintaining the function of a film or in terms of content uniformity.

### Mode for Carrying Out the Invention

The following provides an explanation of embodiments in the present invention. The following embodiments are intended to be exemplary, and are not intended to limit the present invention to only the following embodiments. The present invention can be carried out in various forms provided they do not deviate from the gist thereof.

The compressed composition in the present invention is a compressed composition comprising coated granules containing a physiologically active compound or pharmacologically acceptable salt thereof, and an excipient containing at least one selected from the group consisting of low-substituted hydroxypropylcellulose, lactose and crystalline cellulose, wherein the total amount thereof is 0.5 parts by weight or more in 1 part by weight of the excipient, and the excipient is physically mixed with the coated granules followed by compressing at low pressure.

There are no particular limitations on the method used to physically mix the coated granules with the excipient and compress at low pressure, and the compressed composition in the present invention can be obtained by compressing a mixture of the coated granules and the excipient at low pressure using a device that is generally used in the field of pharmaceutical preparations.
In the device that compresses at low pressure, the coated granules and the excipient may be mixed prior to compressing at low pressure or a mixture thereof may be supplied followed by compressing at low pressure.
A compressed composition obtained by adding the excipient to the coated granules followed by compressing and molding at low pressure is superior in terms of maintaining the function of the film. In addition, the compressed composition is preferably superior in terms of dispersibility of a principal agent or granules containing principal agent (coated granules) together with being superior in terms of maintaining the function of a film and also in terms of the content uniformity of a physiologically active compound or pharmacologically acceptable salt thereof.

The content of the excipient in the compressed composition obtained by physical mixing of an excipient and coated granules is preferably from 0.1 parts by weight to 10 parts by weight, more preferably from 0.2 parts by weight to 5 parts by weight, and even more preferably from 0.2 parts by weight to 2 parts by weight based on 1 part by weight of the coated granules.

The use of the excipient allows the obtaining of a composition that is superior in terms of maintaining the function of a film or superior in terms of content uniformity. Furthermore, the use of a smaller amount of the excipient allows the maintaining of the function of a film being even more superior by further selecting low-substituted hydroxypropylcellulose, lactose and/or crystalline cellulose as the excipient, thereby making it possible to achieve content uniformity and reduce the size of the resulting compressed composition. In addition, the selection of low-substituted hydroxypropylcellulose, lactose and/or crystalline cellulose as the excipient is also superior in terms of compression ability even in the case of tableting using a small amount of the excipient.

The compressed composition in the present invention is a compressed composition comprising coated granules containing a physiologically active compound or pharmacologically acceptable salt thereof, and an excipient containing at least one selected from the group consisting of low-substituted hydroxypropylcellulose, lactose and crystalline cellulose, wherein the total amount thereof is 0.5 parts by weight or more in 1 part by weight of the excipient, and the excipient is coated onto the coated granules followed by compressing at low pressure.

There are no particular limitations on the method used to coat the excipient onto the coated granules, and the excipient can be coated using a device generally used in the field of pharmaceutical preparations.
In the case a film of the excipient formed on the coated granules is coated onto a mixed powder of the coated granules and the excipient, the excipient can be coated directly onto the coated granules by applying or spraying a coating solution.
Examples of solvents for the coating solution include water, ethanol, aqueous ethanol, isopropyl alcohol and acetone, and preferably water, ethanol and aqueous ethanol. The above-mentioned solvent itself may be used as the coating solution in the case of coating the mixed powder, the coating solution may be used that contains an excipient containing at least one selected from the group consisting of low-substituted hydroxypropylcellulose, lactose and crystalline cellulose, or a coating solution may be used that contains a component of the excipients other than low-substituted hydroxypropylcellulose, lactose and crystalline cellulose.
In addition, the excipient can be coated onto the coated granules by coating or spraying a coating solution containing an excipient containing at least one selected from the group consisting of low-substituted hydroxypropylcellulose, lactose and crystalline cellulose onto the coated granules.

The solid content of the excipient coated onto the coated granules relative to the coated granules is preferably from 0.1 parts by weight to 1 part by weight, more preferably from 0.2 parts by weight to 0.8 parts by weight, and even more preferably from 0.4 parts by weight to 0.6 parts by weight based on 1 part by weight of the coated granules. A compressed composition obtained by compressing and molding granules, which are obtained by coating an excipient onto coated granules, at low pressure is superior in terms of dispersibility of a principal agent or granules containing principal-agent (coated granules), and is superior in terms of the content uniformity of a physiologically active compound or pharmacologically acceptable salt thereof.

The excipient used in the present invention contains at least one selected from the group consisting of low-substituted hydroxypropylcellulose, lactose and crystalline cellulose.
The use of low-substituted hydroxypropylcellulose, lactose and/or crystalline cellulose as the excipient allows the obtaining of the desired compressed composition in the present invention by compressing at low pressure.

The term "Compression at low pressure" as used in the present invention refers to compressing at low tableting pressure, and indicates tableting at a pressure being within a substantially lower pressure range compared with tableting pressure in the production of ordinary tablets. Tableting pressure during compression at low pressure in the present invention is preferably from 0.5 kN to 6 kN, more preferably from 1 kN to 5 kN, and even more preferably from 1.5 kN to 5 kN.

The term "Maintaining the function of a film" as used in the present invention is thought to be the result of an effect for preventing destruction of a film on coated granules by excipient during compression, and the excipient contains at least one selected from the group consisting of low-substituted hydroxypropylcellulose, lactose and crystalline cellulose.
Although the excipient may contain a component other than low-substituted hydroxypropylcellulose, lactose and crystalline cellulose, low-substituted hydroxypropylcellulose, lactose and/or crystalline cellulose are preferably contained as main components, provided the excipient has the cushion like action in the present invention.
The content of low-substituted hydroxypropylcellulose, lactose and/or crystalline cellulose in 1 part by weight of the excipient is 0.5 parts by weight or more, preferably 0.8 parts by weight or more and more preferably 0.9 parts by weight or more as the total weight thereof.

In the present invention, the low-substituted hydroxypropylcellulose, lactose and/or crystalline cellulose may each be used alone, or may be used as an arbitrary mixture of these three components. The use of low-substituted hydroxypropylcellulose, lactose and/or crystalline cellulose as the excipient makes it possible to enhance compression ability even in the case of tableting at a low tableting pressure. In addition, low-substituted hydroxypropylcellulose or lactose may also be further arbitrarily used with crystalline cellulose as the excipient.

Examples of low-substituted hydroxypropylcellulose include LH11, LH21, LH22 and LH31 (trade names, Shin-Etsu Chemical Co., Ltd.). In addition, examples of lactose include spray-drying lactose such as SuperTab 11SD (trade name, DMV Fonterra Excipients K.K.) or Dilactose (trade name, Freund Industrial Co., Ltd.). Examples of crystalline cellulose include Ceolus KG-1000, Ceolus PH301, Ceolus PH101, Ceolus UF-711 and Ceolus KG-802 (trade names, Asahi Kasei Chemicals Corp.).

The excipient may contain other components within a range that does not impair the object of the present invention, and examples of components other than low-substituted hydroxypropylcellulose, lactose and/or crystalline cellulose include sugars, sugar-alcohols, glidants, lubricants, binders and disintegrants.

Examples of sugars and sugar-alcohols include mannitol, erythritol, xylitol, sorbitol, trehalose and sucrose. In the present invention, the terms sugar and sugar-alcohol are used with the idea of not including lactose.

Examples of glidants include light anhydrous silicic acid, silicon dioxide and talc.

Examples of lubricants include magnesium stearate, calcium stearate and sodium stearyl fumarate.

The content of components other than low-substituted hydroxypropylcellulose, lactose and/or crystalline cellulose in 1 part by weight of the excipient is 0.5 parts by weight or less, preferably 0.2 parts by weight or less, and more preferably 0.1 part by weight or less.

Excipients may be incorporated by adding each individually or may be used after granulation the components used as excipients in advance.

The coated granules used in the present invention are coated granules containing a physiologically active compound or pharmacologically acceptable salt thereof.
There are no particular limitations on the physiologically active compound provided it is a compound that has a physiological function, and examples thereof include active compounds generally used as active ingredients of pharmaceuticals, diagnostic reagents and the like. In addition, active compounds used as active ingredients of known granular pharmaceuticals can also be used as physiologically active compounds in the present invention.

There are no particular limitations on the pharmacologically acceptable salt provided it is a salt of a physiologically active compound that is commonly used in this field. Examples include salts of a base in the case the physiologically active compound is an acidic compound, and salts of an acid in the case the physiologically active compound is a basic compound. The physiologically active compound may also be a hydrate or solvate.

In the present invention, a benzimidazole compound is a preferable example of a physiologically active compound.
There are no particular limitations on the benzimidazole compound, and examples include rabeprazole, omeprazole, pantoprazole, lansoprazole, nepaprazole, leminoprazole, esomeprazole and 2-[[[4-(2,2-dimethyl-1,3-dioxan-5-yl)methoxy-3,5-dimethylpyridin-2-yl]methyl]sulfinyl]-1H-benzimidazole. There are no particular limitations on pharmacologically acceptable salts of the benzimidazole compounds, and examples thereof include sodium salts, potassium salts, magnesium salts and calcium salts of benzimidazole compounds.
A preferable example of a benzimidazole compound is rabeprazole, and a pharmacologically acceptable salt of rabeprazole can also be used preferably.

The content of the physiologically active compound or pharmacologically acceptable salt thereof can be suitably determined corresponding to that active compound, and is preferably from about 0.04 parts by weight to about 0.13 parts by weight, more preferably from 0.05 parts by weight to 0.12 parts by weight, and even more preferably from 0.8 parts by weight to 0.12 parts by weight based on 1 part by weight of the total weight of the coated granules.

Although there are no particular limitations on the coated granules used in the present invention provided they are granules having a coating layer (film) obtained by a conventionally known method, they are preferably coated granules having a core containing a physiologically active compound or pharmacologically acceptable salt thereof, and a coating in the form of an enteric film, a sustained-release film, a pulsatile-release film, a water-soluble film, a water-insoluble film for bitter taste masking, smell masking or light masking, or a gastric film.
The enteric film, sustained-release film, pulsatile-release film, water-soluble film, water-insoluble film for bitter taste masking, smell masking or light masking, or gastric film can each be suitably selected according to a desired dissolution profile of the active compound.

In the present invention, a compressed composition can be obtained that is resistant to destruction of the film of the coated granules or is superior in terms of content uniformity even after being tableted by physical mixing of the excipient and the coated granules or by coating the excipient onto the coated granules followed by compression at low pressure. Thus, coated granules can be obtained that have as the film thereof a film suitably selected from the group consisting of an enteric film, a sustained-release film, a pulsatile-release film, a water-soluble film, a water-insoluble film for bitter taste masking, smell masking or light masking, or a gastric film.
The enteric film, sustained-release film, pulsatile-release film, water-soluble film, water-insoluble film for bitter taste masking, smell masking or light masking, or gastric film can be coated by coating the film onto a core in accordance with a conventionally known method.
Components used in the film may be selected corresponding to the film application.

In addition, the core may also be a core having a core substance and a principal agent layer that contains a physiologically active compound or pharmacologically acceptable salt thereof and is coated onto the core substance.

The core substance is a substance that serves as a core for adsorbing and coating an active compound, excipients and the like onto the surface thereof in the form of a layer and forming into granules. Although there are no particular limitations on components of the core substance, examples thereof include at least one type of component selected from the group consisting of sugars, sugar-alcohols, celluloses and starches.
Examples of sugars include sucrose and lactose.
Examples of sugar-alcohols include mannitol and erythritol.
Examples of celluloses include crystalline cellulose, while examples of starches include corn starch and potato starch.
The above-mentioned components are preferably used as the core substance since they do not substantially react with the physiologically active compound or pharmacologically acceptable salt thereof.

Examples of the shape of the core substance include a sphere shape, spheroid shape and rugby ball shape since these shapes have a large surface area and superior fluidity. In the case the core is spherical, the mean particle diameter of the core substance is preferably from about 50 µm to about 2000 µm, more preferably from 100 µm to 800 µm, even more preferably from 100 µm to 500 µm and still more preferably from 150 µm to 250 µm.
In the present invention, the mean particle diameter of the core substance can be measured by a method such as a sieving method or optical method.

Commercially available spherical granules may be used for the core substance, and spherical granules may also be used that are obtained by mixing one or more selected from the group consisting of sugars, sugar-alcohols, celluloses and starches followed by granulation and sizing. Examples of commercially available core substances include Nonpareil 101, Nonpareil 103, Nonpareil 105 and Nonpareil 108 (Freund Industrial Co., Ltd.) and Celphere (Asahi Kasei Chemicals Corp.).

From the viewpoint of uniformity of film thicknesses of the principal agent layer and film, the particle size distribution of the core substance is preferably from about 50 µm to about 2000 µm, more preferably from 100 µm to 800 µm, even more preferably from 100 µm to 500 µm and still more preferably from 150 µm to 250 µm. A core substance having a sharp particle size distribution by a sieve may be used for the core substance, and in that case, although a core substance in which particle diameter has been suitably adjusted can be used, a core substance having a median particle diameter of, for example, 100 µm, 200 µm, 300 µm, 400 µm or 500 µm may also be used. In addition, in the case of using a core substance having a particle size distribution of from 150 µm to 250 µm, for example, the core substance can be passed through a sieve to obtain a core substance having a uniform particle size distribution of from 180 µm to 220 µm.
In the present invention, the particle size distribution of the core substance can be measured by a method such as a sieving method or optical method.

In the case of containing a benzimidazole compound as the active compound in the principal agent layer, magnesium oxide may also be contained as an excipient, and ethylcellulose may also be further contained as an excipient. As a result of containing magnesium oxide as an excipient, and preferably magnesium oxide and ethylcellulose as excipients, stability and handling of the benzimidazole compound can be further improved.

The principal agent layer may also contain a water-soluble binder (such as hydroxypropylcellulose, hydroxypropyl methylcellulose or polyvinyl pyrrolidone), a stabilizer (such as magnesium hydroxide, sodium hydroxide or magnesium carbonate), and a lubricant (such as talc or magnesium stearate) and the like.

The principal agent layer can be obtained by coating a coating solution containing an active compound onto the surface of the core substance by coating or spraying.
There are no particular limitations on the method used to coat or spray the coating solution, and a conventional method can be used.
Examples of solvents for the coating solution include water, ethanol, aqueous ethanol, isopropyl alcohol and acetone, and water, ethanol and aqueous ethanol are preferable.
The amount of solid dissolved or dispersed in 1 part by weight of the coating solution is preferably from 0.01 parts by weight to 0.3 parts by weight and more preferably from 0.05 parts by weight to 0.2 parts by weight.

The principal agent layer may also be coated with enteric film so as to prevent dissolution of the principal agent in the stomach.
Examples of enteric polymers contained in the enteric film include hydroxypropyl methylcellulose phthalate (trade names: HP-55, HP-55S, HP-50, Shin-Etsu Chemical Co., Ltd.), hydroxypropyl methylcellulose acetate succinate (trade name: Shin-Etsu AQOAT, Shin-Etsu Chemical Co., Ltd.), methacrylic acid methyl methacrylate copolymer (trade name: Eudragit L100, Eudragit S100, Rohm Pharma GmbH), methacrylic acid ethyl acrylate copolymer (trade name: Eudragit L100-55, Eudragit L-30D55, Rohm Pharma GmbH), carboxymethyl ethylcellulose (trade name: CMEC, Freund Industrial Co., Ltd.), polyvinyl alcohol acetate phthalate (trade name: Opa-Dry Enteric, Colorcon Japan LLC), and cellulose acetate phthalate (trade name: CAP, Wako Pure Chemical Industries, Ltd.).

An enteric film can be coated onto the principal agent layer by coating or spraying a coating solution of the enteric film containing at least an enteric polymer onto the principal agent layer.
Examples of solvents for the coating solution of the enteric film include water, ethanol, aqueous ethanol, isopropyl alcohol and acetone, and water, ethanol and aqueous ethanol are preferable.
The amount of solid dissolved or dispersed in 1 part by weight of the coating solution of the enteric film is preferably from 0.01 parts by weight to 0.3 parts by weight and more preferably 0.2 parts by weight or less.

Enteric polymer may be contained from about 0.6 parts by weight to about 0.95 parts by weight, preferably 0.75 parts by weight or more, and more preferably 0.8 parts by weight or more based on 1 part by weight of the total weight of the enteric film. In addition, it may also be contained at 0.9 parts by weight or less.

The enteric film may be contained from about 0.3 parts by weight to about 0.8 parts by weight, preferably from 0.4 parts by weight to 0.7 parts by weight and even more preferably from 0.45 parts by weight to 0.6 parts by weight based on 1 part by weight of the total weight of the coated granules.

The enteric film may also contain a lubricant, glidant, plasticizer and the like.

Examples of lubricants include magnesium stearate, calcium stearate, sodium stearyl fumarate, talc, synthetic magnesium silicate, carnauba wax, hydrogenated oil and microcrystalline wax.
Examples of glidants include light anhydrous silicic acid and titanium oxide.

The sustained-release film, pulsatile-release film, water-soluble film, water-insoluble film for bitter taste masking, smell masking or light masking and gastric film can also be provided by coating onto the principal agent layer in the same manner as the enteric film.
Conventionally known components, which are known to be able to demonstrate sustained-release, pulsatile-release, bitter taste masking and gastric dissolution when used as a granule film are coated for the components of the coating solution of the sustained-release film, pulsatile-release film, water-soluble film, water-insoluble film for bitter taste masking, smell masking or light masking and gastric film.

In the case the active compound is an acidic compound, it may have one or more intermediate layers between the principal agent layer and the enteric film, sustained-release film, pulsatile-release film, water-soluble film, water-insoluble film for bitter taste masking, smell masking or light masking and gastric film.
The intermediate layers may also contain a lubricant, water-insoluble polymer, water-soluble polymer and the like.
Intermediate layer might prevent contacting with active compound and enteric polymer being an acidic substance, thereby making it possible to further improve stability of the active compound.

Examples of water-insoluble polymers include ethylcellulose (trade name: Ethocel, Dow Chemical Co.), cellulose acetate (Eastman Chemical Co.), carboxymethyl ethylcellulose (trade name: CMEC, Freund Industrial Co., Ltd.), aminoalkyl methacrylate copolymer RS (trade name: Eudragit RS, Rohm Pharma GmbH), crospovidone (trade name: Kollidon CL, BASF Co., Ltd.), wax, shellac (Japan Shellac Industries, Ltd.), vinyl acetate resin, polyvinylacetal diethylaminoacetate (trade name: AEA, Sankyo Co., Ltd.), acrylic acid ethylmethylmethacrylate copolymer (trade name: Eudragit NE, Rohm Pharma GmbH), carboxymethylcollulose (trade name: Carmellose, Nichirin Chemical Industries, Ltd.), low-substituted hydroxypropylcellulose (trade name: L-HPC, Shin-Etsu Chemical Co., Ltd.) and crystalline cellulose (trade name: Avicel, Ceolus, Asahi Kasei Chemicals Corp.).

Examples of water-soluble polymers include hydroxypropylcellulose (Nippon Soda Co., Ltd.), hydroxypropyl methylcellulose (trade name: TC-5, Shin-Etsu Chemical Co., Ltd.), methylcellulose (trade name: Metolose, Shin-Etsu Chemical Co., Ltd.), polyvinyl alcohol (trade name: Gohsenol, Nippon Goseikagaku Kogyo K.K.), copolyvidone (trade name: Kollidon VA64, BASF Co., Ltd.; Plasdone S-630, ISP Japan Ltd.), polyvinyl pyrrolidone (trade name: Kollidon, BASF Co., Ltd.; Plasdone, ISP Japan Ltd.), and polyvinyl alcohol- polyethylene glycol graft copolymer (trade name: Kollicoat IR, BASF Co., Ltd.).

The intermediate layer may also contain a lubricant (such as magnesium stearate, calcium stearate, sodium stearyl fumarate, talc, synthetic magnesium silicate, carnauba wax, hydrogenated oil or microcrystalline wax).

The coated granules may have two or more intermediate layers, and in the case of having two or more intermediate layers, the two or more intermediate layers can be coated onto the principal agent layer by preparing an intermediate layer coating solution containing the same or different components, and coating or spraying the intermediate layer coating solution onto the principal agent layer.
In the case of having two or more intermediate layers, for example, a layer containing a water-insoluble polymer and water-soluble polymer, and as necessary, a lubricant such as magnesium stearate can be provided for the first intermediate layer, and a layer containing a water-insoluble polymer such as crospovidone can be provided for the second intermediate layer.

Examples of solvents for the intermediate layer coating solution include water, ethanol, aqueous ethanol, isopropyl alcohol and acetone, and water, ethanol and aqueous ethanol are preferable.
The amount of solid dissolved or dispersed in 1 part by weight of the intermediate layer coating solution is preferably from 0.01 parts by weight to 0.3 parts by weight and more preferably 0.2 parts by weight or less.

The intermediate layer is contained from about 0.1 parts by weight to about 0.5 parts by weight, preferably from 0.15 parts by weight to 0.45 parts by weight and more preferably from 0.2 parts by weight to 0.35 parts by weight based on 1 part by weight of the total weight of the coated granules.

The coated granules in the present invention may further have a coated lubricant, or a lubricant may be further coated onto a film.

The granules in the present invention can be produced in the manner described below.
A first coating solution is prepared by dissolving or dispersing a benzimidazole compound in ethanol or purified water and the like, and the first coating solution is coated or sprayed onto a core substance followed by drying to obtain a core coated with a principal agent layer. Next, a second coating solution is prepared by dissolving or dispersing an enteric polymer in ethanol or purified water and the like, and the second coating solution is coated or sprayed onto the principal agent layer of the core followed by drying to obtain coated particles having an enteric film.

One or more intermediate layers containing a water-insoluble polymer and water-soluble polymer may also be coated between the first coating layer and the second coating layer as necessary.

Examples of means used to spray the coating solution include a centrifugal fluidized granulation coating device, fluidized bed granulation coater and wurster fluidized bed granulation coater.

The mean particle diameter of the coated granules may be, for example, from 200 µm to 1000 µm, preferably from 300 µm to 800 µm, and more preferably from 400 µm to 650 µm.

The compressed composition in the present invention can be formed into a tablet, and although there are no particular limitations on the tablet, an example thereof is a matrix tablet.
There are no particular limitations on the tableting machine used to produce a tablet in the present invention, and a conventionally known tableting machine can be used.
In the present invention, a matrix tablet refers to a tablet of a form in which principal agent granules are uniformly dispersed in the tablet.

In the present invention, a compressed composition obtained in the form of a matrix tablet can also be used as an inner core for a dry coated tablet.
In the present invention, a dry coated tablet refers to a tablet of a form in which a principal agent layer is localized in the tablet. The compressed composition in the present invention can also be used as a part of the inner core for a dry coated tablet since it is superior in terms of maintaining the function of a film.

In the present invention, the compressed composition can be produced by a method comprising physically mixing coated granules containing a physiologically active compound or pharmacologically acceptable salt thereof with excipient containing at least one selected from the group consisting of low-substituted hydroxypropylcellulose, lactose and crystalline cellulose, the total amount thereof in 1 part by weight of the excipient being 0.5 parts by weight or more, or coating the excipient onto the coated granules, followed by compression at low pressure. In addition, the compressed composition can also be obtained by mixing with the excipient when the excipient is coated onto the coated granules and the resultant mixture is compressed at low pressure.
Adry coated tablet containing the resulting matrix tablet as the inner core thereof can be obtained by covering the resulting matrix tablet with an outer layer powder followed by compression.
Examples of the outer layer powder include known components used in the outer layer of dry coated tablets, including sugars (such as sucrose or lactose), sugar-alcohols (such as mannitol, xylitol or sorbitol), binders (such as hydroxypropylcellulose, hydroxypropyl methylcellulose, polyvinyl pyrrolidone, crystalline cellulose or low-substituted hydroxypropylcellulose), disintegrants (such as crospovidone, croscarmellose sodium or carmellose calcium) and lubricants (such as magnesium stearate, calcium stearate or sodium stearyl fumarate).

The pressure in compression of the outer layer powder is preferably from 2 kN to 14 kN, more preferably from 3 kN to 12 kN and even more preferably from 4 kN to 9 kN.
As a result of compression by controlling the pressure to within the above-mentioned ranges, a dry coated tablet having superior stability can be obtained in the form of a dry coated tablet containing the compressed composition in the present invention without breaking the film for the coated granules in the compressed composition in the inner core.

The filled amount of the matrix tablet contained in the inner layer for the dry coated tablet is preferably from 0.1 parts by weight to 0.8 parts by weight, more preferably from 0.15 parts by weight to 0.7 parts by weight, and even more preferably from 0.2 parts by weight to 0.5 parts by weight based on 1 part by weight of the total weight of the dry coated tablet.

In the case of using general principal agent granules, although there are cases in which the film of the coated granules may be damaged during tableting or stability and efficacy of the physiologically active compound may be impaired during tableting process, by using a matrix tablet that has been compressed at low pressure using the excipients in the present invention as the inner core, the film remains stable even during tableting and a dry coated tablet can be produced without damage of the function of the film.

The tablet diameter of the compressed composition in the present invention is preferably from 5 mm to 13 mm, more preferably from 6 mm to 10 mm and even more preferably from 7 mm to 9 mm.

The compressed composition in the present invention can also be used as an orally disintegrating tablet.

### Examples

Although the following provides a more detailed explanation of the present invention by listing examples thereof, these examples are intended to be exemplary and are not to be interpreted as limiting the present invention.

### (Measurement of Acid Resistance)

The resulting compressed composition was added into 0.1 N HCl followed by measurement of the concentration of a physiologically active compound solution after two hours.
The resulting dissolved amount of the active compound (%) was then measured and showed as the measured value of acid resistance.

### (Measurement of Content Uniformity)

Content uniformity was evaluated based on a content uniformity test as described in the Japanese Pharmacopoeia Fifteenth Edition.

### [Production Example 1]

### (Production of Coated Granules)

28.56 kg of a core substance in the form of Nonpareil 108 (trade name: Freund Industrial Co., Ltd.) were coated with a coating solution obtained by dissolving 17 kg of rabeprazole sodium and 4.08 kg of ethylcellulose (trade name: Ethocel, Dow Chemical Co.) in 187.93 kg of ethanol anhydrous and further dispersing 5.44 kg of magnesium oxide (Tomita Pharmaceutical Co., Ltd.) therein using a wurster fluidized bed granulation coater (trade name: GPCG-SPC, Powrex Corp.) followed by drying to obtain granules.
Next, 55.08 kg of the above-mentioned granules were coated with a coating solution obtained by dissolving 4.08 kg of ethylcellulose (trade name: Ethocel, Dow Chemical Co.) and 58.48 kg of hydroxypropylcellulose (trade name: HPC-L, Nippon Soda Co., Ltd.) in 1370.41 kg of ethanol anhydrous and further dispersing 27.20 kg of magnesium stearate (Mallinckrodt Inc.) therein followed by drying to obtain intermediate layer coated granules.
Moreover, 75.14 kg of the above-mentioned intermediate layer coated granules were coated with a coating solution obtained by dissolving 68 kg of hydroxypropyl methylcellulose phthalate (trade name: HP-55S, Shin-Etsu Chemical Co., Ltd.) and 6.80 kg of diacetylated monoglyceride (trade name: Myvacet, Kerry Inc.) in 1632.70 kg of 80% aqueous ethanol and further dispersing 6.46 kg of talc (trade name: Crown Talc, Matsumura Sangyo Co., Ltd.) and 3.40 kg of titanium oxide (Merck & Co.) therein followed by drying to obtain enteric coated granules.
The resulting enteric coated granules were mixed with 1.70 kg of light anhydrous silicic acid (Nippon Aerosil Co., Ltd.) and 1.70 kg of talc (trade name: Hi-Filler, Matsumura Sangyo Co., Ltd.) to obtain coated granules for tableting.

### [Production Example 2]

### (Production of Coated Granules)

3570 g of a core substance in the form of Nonpareil 108 (trade name: Freund Industrial Co., Ltd.) were coated with a coating solution obtained by dissolving 8500 g of rabeprazole sodium and 2040 g of ethylcellulose (trade name: Ethocel, Dow Chemical Co.) in 93964 g of ethanol anhydrous and further dispersing 2720 g of magnesium oxide (Tomita Pharmaceutical Co., Ltd.) therein using a wurster fluidized bed granulation coater (trade name: GPCG-SPC, Powrex Corp.) followed by drying to obtain granules.
Next, 8415 g of the above-mentioned granules were coated with a coating solution obtained by dissolving 892.5 g of ethylcellulose (trade name: Ethocel, Dow Chemical Co.) and 5355 g of hydroxypropylcellulose (trade name: HPC-L, Nippon Soda Co., Ltd.) in 126028 g of ethanol anhydrous and further dispersing 2507.5 g of magnesium stearate (Mallinckrodt Inc.) therein followed by drying to obtain intermediate layer coated granules.
Moreover, 8585 g of the above-mentioned intermediate layer coated granules were coated with a coating solution obtained by dissolving 8542.5 g of hydroxypropyl methylcellulose phthalate (trade name: HP-55S, Shin-Etsu Chemical Co., Ltd.) and 850 g of diacetylated monoglyceride (trade name: Myvacet, Kerry Inc.) in 193022 g of 80% aqueous ethanol and further dispersing 403.8 g of talc (trade name: Crown Talc, Matsumura Sangyo Co., Ltd.) and 212.5 g of titanium oxide (Merck & Co.) therein followed by drying to obtain enteric coated granules.
The resulting enteric coated granules were mixed with 191.3 g of light anhydrous silicic acid (Nippon Aerosil Co., Ltd.) and 191.3 g of talc (trade name: Hi-Filler, Matsumura Sangyo Co., Ltd.) to obtain coated granules for tableting.

### [Production Example 3]

### (Coating of Excipient onto Coated Granules)

3570 g of a core substance in the form of Nonpareil 108 (trade name: Freund Industrial Co., Ltd.) were coated with a coating solution obtained by dissolving 8500 g of rabeprazole sodium and 2040 g of ethylcellulose (trade name: Ethocel, Dow Chemical Co.) in 93964 g of ethanol anhydrous and further dispersing 2720 g of magnesium oxide (Tomita Pharmaceutical Co., Ltd.) therein using a wurster fluidized bed granulation coater (trade name: GPCG-SPC, Powrex Corp.) followed by drying to obtain granules.
Next, 8523 g of the above-mentioned granules were coated with a coating solution obtained by dissolving 873.3 g of ethylcellulose (trade name: Ethocel, Dow Chemical Co.) and 5240.3 g of hydroxypropylcellulose (trade name: HPC-L, Nippon Soda Co., Ltd.) in 123323 g of ethanol anhydrous and further dispersing 2453.9 g of magnesium stearate (Mallinckrodt Inc.) therein followed by drying to obtain intermediate layer coated granules.
Moreover, 8391 g of the above-mentioned intermediate layer coated granules were coated with a coating solution obtained by dissolving 7984.8 g of hydroxypropyl methylcellulose phthalate (trade name: HP-55S, Shin-Etsu Chemical Co., Ltd.) and 794.4 g of diacetylated monoglyceride (trade name: Myvacet, Kerry Inc.) in 180421 g of 80% aqueous ethanol and further dispersing 377.6 g of talc (trade name: Crown Talc, Matsumura Sangyo Co., Ltd.) and 198.7 g of titanium oxide (Merck & Co.) therein followed by drying to obtain enteric coated granules.
In addition, 82.3 g of hydroxypropylcellulose (trade name: HPC-L, Nippon Soda Co., Ltd.) were dissolved in 2057 g of ethanol anhydrous and sprayed onto a powder layer obtained by mixing 1007.2 g of the above-mentioned enteric coated granules, 299.4 g of crystalline cellulose (Ceolus KG-1000, Asahi Kasei Chemicals Corp.) and 201.1 g of low-substituted hydroxypropylcellulose (L-HPC (LH11), Shin-Etsu Chemical Co., Ltd.) using a wurster fluidized bed granulation coater (trade name: GPCG-SPC, Powrex Corp.) to obtain granules coated with excipients.

**[Table 1]**

| | Production Example 1 | Production Example 2 | Production Example 3 |
|---|---|---|---|
| Nonpareil 108 | 4.2 | 4.2 | 4.2 |
| Magnesium oxide | 0.8 | 3.2 | 3.2 |
| Rabeprazole sodium | 2.5 | 10 | 10 |
| Ethylcellulose | 0.6 | 2.4 | 2.4 |
| Ethanol anhydrous | q.s. | q.s. | q.s. |
| Ethylcellulose | 1.4 | 2.1 | 2.1 |
| Hydroxypropylcellulose | 8.6 | 12.6 | 12.6 |
| Magnesium stearate | 4 | 5.9 | 5.9 |
| Ethanol anhydrous | q.s. | q.s. | q.s. |
| Hydroxypropyl methylcellulose phthalate | 20 | 40.2 | 40.2 |
| Diacetylated monoglyceride | 2 | 4 | 4 |
| Talc | 1.9 | 1.9 | 1.9 |
| Titanium oxide | 1 | 1 | 1 |
| Ethanol | q.s. | q.s. | q.s. |
| Purified water | q.s. | q.s. | q.s. |
| Light anhydrous silicic acid | 0.5 | 0.9 | - |
| Talc | 0.5 | 0.9 | - |
| Crystalline cellulose | - | | 18.6 |
| Low-substituted hydroxypropylcellulose | - | - | 12.5 |
| Hydroxypropylcellulose | - | - | 5.1 |
| Ethanol anhydrous | - | - | q.s. |

Nonpareil having a sharp particle size distribution obtained by passing through a sieve was used for the Nonpareil 108 of Production Examples 2 and 3.

### [Example 1]

75 g of crystalline cellulose (Ceolus KG-1000, Asahi Kasei Chemicals Corp.), 223.5 g of lactose (SuperTab 11SD, DMV Fonterra Excipients K.K.) and 1.5 g of magnesium stearate (Mallinckrodt Inc.) were added to 1000 g of the coated granules obtained in Production Example 1 and mixed. The resulting mixture was tableted with a rotary tableting machine (φ7 mm, tablet weight: 100 mg, compression pressure: 6 kN) to obtain a compressed composition. The results of acid resistance of the resulting compressed composition are shown in Table 2.

**[Table 2]**

| | Ex. 1 | Ref. Ex. 1 | Ref. Ex. 2 | Ref. Ex. 3 | Ref. Ex. 4 |
|---|---|---|---|---|---|
| Excipients (223.5 each) | Lactose | Sucrose | Anhydrous dibasic calcium phosphate | Cross carmellose sodium | Isomalt |
| Crystalline cellulose | 75 | 75 | 75 | 75 | 75 |
| Magnesium stearate | 1.5 | 1.5 | 1.5 | 1.5 | 1.5 |
| Compression pressure (kN) | 5 | 6 | 5 | 5 | 5.5 |
| Acid resistance (%) | 9.6 | 24.4 | 13.1 | 19.5 | 34.9 |

The incorporated amounts are indicated for the case of 1000 parts by weight of coated granules.

### [Reference Examples 1 to 4]

Compressed compositions were obtained in the same manner as Example 1 with the exception of changing the lactose used in Example 1 to each excipients shown in Table 2 and by the compression pressures shown in Table 2 .
The excipients used in each of the reference examples were as shown below.
Sucrose spherical granules: Nonpareil 103, Freund Industrial Co., Ltd.
Anhydrous dibasic calcium phosphate: Fujicalin SG, Fuji Chemical Industry Co., Ltd.
Crosscarmellose sodium: Ac-Di-Sol, Dainippon Sumitomo Pharma Co., Ltd.
Isomalt: Galen IQ 981, Beneo-Palatinit GmbH

### [Example 2]

250 g of crystalline cellulose (Ceolus KG-1000, Asahi Kasei Chemicals Corp.), 247.5 g of lactose (SuperTab, DMV Fonterra Excipients K.K.) and 2.5 g of magnesium stearate (Mallinckrodt Inc.) were added to 1000 g of the coated granules obtained in Production Example 1 and mixed. The resulting mixture was compressed using a rotary tableting machine (φ8 mm, tablet weight: 150 mg, compression pressure: 1.7 kN) to obtain a compressed composition. The results of acid resistance of the resulting compressed composition are shown in Table 3.

### [Example 3]

75 g of crystalline cellulose (Ceolus KG-1000, Asahi Kasei Chemicals Corp.), 50 g of low-substituted hydroxypropylcellulose (L-HPC (LH21), Shin-Etsu Chemical Co., Ltd.), 118.8 g of lactose (SuperTab, DMV Fonterra Excipients K.K.) and 1.3 g of magnesium stearate (Mallinckrodt Inc.) were added to 500 g of the coated granules obtained in Production Example 1 and mixed. The resulting mixture was compressed with a rotary tableting machine (φ8 mm, tablet weight: 150 mg, compression pressure: 1.7 kN) to obtain a compressed composition. The results of acid resistance of the resulting compressed composition are shown in Table 3.

**[Table 3]**

| | Example 2 | Example 3 |
|---|---|---|
| Crystalline cellulose | 250 | 150 |
| Low-substituted hydroxypropylcellulose | - | 100 |
| Lactose | 247.5 | 237.5 |
| Light anhydrous silicic acid | -- | 10 |
| Magnesium stearate | 2.5 | 2.5 |
| Compression pressure (kN) | 1.7 | 1.7 |
| Acid resistance (%) | 5.7 | 2.5 |

The incorporated amounts are indicated for the case of 1000 parts by weight of coated granules.

### [Example 4]

150 g of crystalline cellulose (Ceolus KG-1000, Asahi Kasei Chemicals Corp.), 100 g of low-substituted hydroxypropylcellulose (L-HPC (LH21), Shin-Etsu Chemical Co., Ltd.), 237.5 g of lactose (SuperTab, DMV Fonterra Excipients K.K.), 10 g of light anhydrous silicic acid (Sylysia 320, Fuji Silysia Chemical Ltd.) and 2.5 g of magnesium stearate (Mallinckrodt Inc.) were added to 1000 g of the coated granules obtained in Production Example 1 and mixed. The resulting mixture was compressed with a rotary tableting machine (φ7.5 mm, tablet weight: 130 mg, compression pressure: 2.2 kN) to obtain a compressed composition. The results of acid resistance and content uniformity of the resulting compressed composition are shown in Table 4.

### [Example 5]

A compressed composition was obtained in the same manner as Example 8 using the excipients shown in Table 4 and tableting by a compression pressure of 1.8 kN. The results of acid resistance and content uniformity of the resulting compressed composition are shown in Table 4. In Table 4, a judgment of acceptable meant that it was acceptable in a content uniformity test.

**[Table 4]**

| | Example 4 | | Example 5 | |
|---|---|---|---|---|
| Crystalline cellulose | 150 | | 250 | |
| Low-substituted hydroxypropylcellulose | 100 | | - | |
| Lactose | 237.5 | | 237.5 | |
| Light anhydrous silicic acid | 10 | | 10 | |
| Magnesium stearate | 2.5 | | 2.5 | |
| Compression pressure (kN) | 2.2 | | 1.8 | |
| Acid resistance (%) | 4.1 | | 2.4 | |
| Content uniformity (%) | Start | End | Start | End |
| Average | 103.9 | 103.7 | 109.2 | 93.9 |
| Max. | 106.4 | 106.6 | 112 | 98.6 |
| Min. | 100.8 | 101.2 | 104.8 | 86.8 |
| RSD | 1.5 | 1.7 | 2 | 3.7 |
| Acceptance Value | 6.3 | 6.4 | 13.1 | 12.9 |
| Judgment | Acceptable | Acceptable | Acceptable | Acceptable |

The incorporated amounts are indicated for the case of 1000 parts by weight of coated granules.

### [Example 6]

27.7 g of crystalline cellulose (Ceolus KG-1000, Asahi Kasei Chemicals Corp.), 18.5 g of low-substituted hydroxypropylcellulose (L-HPC (LH21), Shin-Etsu Chemical Co., Ltd.), 45.7 g of lactose (SuperTab, DMV Fonterra Excipients K.K.) and 0.5 g of magnesium stearate (Mallinckrodt Inc.) were added to 307.7 g of the coated granules obtained in Production Example 1 and mixed. This was compressed using a rotary tableting machine (φ7 mm, tablet weight: 100 mg, compression pressure: 4 kN) to obtain a compressed composition. The results of acid resistance of the resulting compressed composition are shown in Table 5.
Although compressed compositions compressed in the same manner as Example 6 were attempted to be obtained for the reference examples, they were unable to be compressed at a compression pressure of 4 kN.

**[Table 5]**

| | Ref. Ex. 5 | Ref. Ex. 6 | Ref. Ex. 7 | Ref. Ex. 8 | Ex. 6 |
|---|---|---|---|---|---|
| Crystalline cellulose | 75 | 75 | 75 | 75 | 90 |
| Crosscarmellose sodium | 223.5 | - | - | - | - |
| Sucrose | - | 223.5 | -- | - | - |
| Anhydrous dibasic calcium phosphate | - | - | 223.5 | - | - |
| Isomalt | - | - | - | 223.5 | - |
| Low-substituted hydroxypropylcellulose | - | - | - | - | 60 |
| Lactose | - | - | - | - | 148.5 |
| Magnesium stearate | 1.5 | 1.5 | 1.5 | 1.5 | 1.5 |
| Acid resistance (%) | × | × | × | × | 6.9 |

The incorporated amounts are indicated for the case of 1000 parts by weight of coated granules. "x" indicates that it was not possible to compress at 4 kN.

### [Example 7]

10 g of crystalline cellulose (Ceolus KG-1000, Asahi Kasei Chemicals Corp.), 6.67 g of low-substituted hydroxypropylcellulose (L-HPC (LH11), Shin-Etsu Chemical Co., Ltd.), 12.5 g of lactose (SuperTab, DMV Fonterra Excipients K.K.), 4 g of light anhydrous silicic acid (Sylysia 320, Fuji Silysia Chemical Ltd.) and 0.17 g of magnesium stearate (Mallinckrodt Inc.) were added to 166.7 g of the coated granules obtained in Production Example 1 and mixed. The resulting mixture was compressed using a rotary tableting machine (φ7 mm, tablet weight: 100 mg, compression pressure: 4 kN) to obtain a compressed composition. The results of acid resistance of the resulting compressed composition are shown in Table 6.

### [Examples 8 to 12]

Compressed compositions were obtained by using the excipients described in Table 6 and tableting in the same manner as Example 7. The results of acid resistance of the resulting compressed compositions are shown in Table 6.

**[Table 6]**

| | Ex. 7 | Ex. 8 | Ex. 9 | Ex. 10 | Ex. 11 | Ex. 12 |
|---|---|---|---|---|---|---|
| Crystalline cellulose | 60 | 89.7 | 150 | 180 | 300 | 599.7 |
| Low-substituted hydroxypropylcellulose | 40 | 60 | 99.8 | 120 | 200 | 400.3 |
| Lactose | 75 | 122.2 | 217.6 | 264.8 | 455 | 929.5 |
| Light anhydrous silicic acid | 24 | 26 | 30 | 32 | 40 | 60 |
| Magnesium stearate | 1 | 1.5 | 2.5 | 3 | 5 | 10 |
| Acid resistance (%) | 10.1 | 3.4 | 1.1 | 1.4 | 3.2 | 5 |
| Coated granules: Excipients | 10:2 | 10:3 | 10:5 | 10:6 | 10:10 | 10:20 |

The incorporated amounts are indicated for the case of 1000 parts by weight of coated granules.

### [Examples 13 and 14]

Compressed compositions were obtained by compression of the coated granules obtained in Production Example 3 using a rotary tableting machine (φ7 mm, tablet weight: 123.7 mg, compression pressure: 3.5 kN or 5 kN). The results of content uniformity of the resulting compressed compositions are shown in Table 7.

**[Table 7]**

| | Example 13 | | Example 14 | |
|---|---|---|---|---|
| Compression pressure (kN) | 3.5 | | 5 | |
| Content uniformity (%) | Start | End | Start | End |
| Average | 99.3 | 99.7 | 100.2 | 98.3 |
| Max. | 101.4 | 101.9 | 104.8 | 100.4 |
| Min. | 97.1 | 97.8 | 95.6 | 96.4 |
| RSD | 1.6 | 1.3 | 2.6 | 1.4 |
| Acceptance Value | 3.7 | 3.1 | 6.2 | 3.5 |
| Judgment | Acceptable | Acceptable | Acceptable | Acceptable |

### Industrial Applicability

The compressed composition in the present invention is a composition that is superior in terms of maintaining the function of a film or content uniformity, and has industrial applicability in fields such as pharmaceuticals.

## Claims

1. Acompressed composition, comprising:
coated granules containing a physiologically active compound or pharmacologically acceptable salt thereof, and an excipient, wherein
the excipient contains at least one selected from the group consisting of low-substituted hydroxypropylcellulose, lactose and crystalline cellulose, and the total amount thereof is 0.5 parts by weight or more in 1 part by weight of the excipient, and
the excipient is physically mixed with or coated onto the coated granules followed by compressing at low pressure.

2. The compressed composition according to claim 1, wherein the excipient is physically mixed with the coated granules followed by compressing at a low pressure from 0.5 kN to 6 kN.

3. The compressed composition according to claim 1, wherein the excipient is coated onto the coated granules followed by compressing at a low pressure from 0.5 kN to 6 kN.

4. The compressed composition according to any of claims 1 to 3, wherein the coated granules have:
a core containing a physiologically active compound or pharmacologically acceptable salt thereof, and
an enteric film, a sustained-release film, a pulsatile-release film, a gastric film, a water-insoluble film for bitter taste masking, smell masking or light masking, or a water-soluble film coated onto the core.

5. The compressed composition according to any of claims 1 to 4, wherein the core has:
a core substance, and
a principal agent layer coated onto the core substance and containing a physiologically active compound or pharmacologically acceptable salt thereof.

6. The compressed composition according to any of claims 1 to 5, wherein the physiologically active compound is a benzimidazole compound.

7. The compressed composition according to any of claims 1 to 6, wherein the excipient further contains a sugar or a sugar-alcohol.

8. The compressed composition according to any of claims 1 to 7, wherein the excipient further contains a glidant.

9. The compressed composition according to any of claims 1 to 8, wherein the excipient further contains a lubricant.

10. The compressed composition according to claim 2, containing 1 part by weight of the coated granules and from 0.1 parts by weight to 10 parts by weight of the excipient.

11. The compressed composition according to claim 3, containing 1 part by weight of the coated granules and from 0.1 parts by weight to 1 part by weight of the excipient.

12. The compressed composition according to any of claims 1 to 11, which is a matrix tablet.

13. A dry coated tablet containing the matrix tablet according to claim 12 as the inner core thereof.

14. A method for producing a compressed composition, comprising the steps of:
physically mixing an excipient with coated granules containing a physiologically active compound or pharmacologically acceptable salt thereof, or coating an excipient onto coated granules, and
compressing the resulting product at low pressure, wherein
the excipient contains at least one selected from the group consisting of low-substituted hydroxypropylcellulose, lactose and crystalline cellulose, and the total amount thereof is 0.5 parts by weight or more in 1 part by weight of the excipient.
